# EUROPEAN PATENT SPECIFICATION

(11) **EP 1 386 629 B1**
(45) Date of publication and mention of the grant of the patent: **25.08.2010**
(21) Application number: 03025749.7
(22) Date of filing: 11.04.1995
(51) Int. Cl.: A61M 13/00, A61M 16/10

(54) **Method for conditioning gas**
Methode zur Konditionierung von Gas
Procédé de conditionement de gaz

(43) Date of publication of application: 04.02.2004
(62) Divisional of application: 95916308.0
(73) Proprietor: Lexion Medical, LLC, St. Paul, MN 55112-2750 (US)
(72) Inventor: Ott, Douglas E., Macon GA 31210 (US); sCHAEFER, John, F., Macon GA 31210 (US); Gray, Robert, I., Macon GA 31201 (US)
(74) Representative: Schäfer, Horst

(56) References cited:
- WO-A-91/19527
- US-A- 4 121 583
- US-A- 4 825 863
- US-A- 5 006 109
- US-A- 5 246 419
- US-A- 5 383 447

## Description

### BACKGROUND OF THE INVENTION

### FIELD OF THE INVENTION

This invention relates to altering the temperature, humidity and the presence of physical and biological contaminants of gases used to inflate body cavities prior to and during medical procedures. More specific-ally, it relates to a compact device for, and method of, heating, humidifying and filtering insufflation gases at a point immediately prior to passage of the gases into the patient.

### BACKGROUND ART

From the beginning of laparoscopic surgical procedures some twenty years ago, it has been assumed that the condition of gases used to inflate body cavities were physiologically and pathologically benign. While the importance and use of temperature and moisture conditioning of anesthesia gases has been well known, until recently little attention had been given to the particulate, temperature and/or humidity condition of insufflation gases used to create a pneumoperitoneum.

As commonly used insufflation gas is carbon dioxide which is typically provided as a liquid in compressed gas cylinders. The pressure in these cylinders, when at equilibrium with ambient environment of 20°C, is 57 atmosphere (5740 KPa). The carbon dioxide gas is typically provided to the surgical site at a pressure of 15mmHg via an adjustable, throttling pressure regulator and flow controller called an insufflator. Many models of insufflators are available such as the Storz Model 26012 (Karl Storz Endoscopy-America Inc., Culver City, California). In general, insufflators do not filter, control the temperature of or humidify the gas.

When the insufflator provides gas flows of various magnitudes, typically 1 to 10 liters per minute, it must reduce the gas pressure of the gas from the cylinder pressure from about 57 atmosphere to approximately 1 atmosphere. Such a process is called "throttling," which causes the gas to be cooled via a thermodynamic process known as Joule-Thompson cooling (*see*, for example, Y.A. Congel and M.A. Bole,% "Thermodynamics: An Engineering Approach," McGraw-Hill, (1988)).

With the carbon dioxide as the insulation gas, Joule-Thompson cooling can reduce the gas temperature as much as 50° to 70°C, depending on gas mass flow rates. The fortuitous, large difference in heat capacities of the insufflator metal hardware (large) and the CO₂ gas stream (small) permits the gas stream to be reheated to approximately operating room ambient temperature (around 20°C) before the gas enters the patient. In the case of large gas flow% this unplanned and uncontrolled reheating effect could be incomplete and the insufflator gas Could leave the insufflator apparatus at temperatures considerably less than the ambient temperature of approximately 20°C. In any case, insufflator gas cannot reach a temperature higher than this ambient temperature, and hence, the insufflator gas enters the patient at a temperature substantially less (at least 17°C less) than the patient's physiological core or approximately 37°C (Ott, D.E., *J. Laparoendosc. Surg.*, 1:127-131 (1191)).

Newly developed insufflators and ancillary devices have recognized this problem and have attempted to correct it by adding heat to the gas stream before it enters the delivery system which directs the gas to the trocars (*see*, for example, Computerized High Flow Insufflator (Snowden-Pencer, Inc., Tucker, Georgia) and How-Thenne (Wisap U.S.A., Tomball, Texas)). This method is thermodynamically unsound because it fails to recognize the thermal-capacity mismatch between the flowing gas stream and the gas delivery system between the insufflator and the trocar incision point at the abdomen, even when the delivery system is only 6 to 10 feet of polymer tubing. In addition, this method overlooks the active heat transfer that occurs between the gas stream and the ambient temperature gas delivery tubing. Because of these thermal conditions, the temperature of any gas preheated at or in the insufflator itself will return to approximately the ambient temperature after flowing as little as four (4) feet after leaving the insufflator.

U.S. Patent No. 5,006,109 (Douglas et al.) relocates the temperature sensor to the point of gas administration, but this relocation does not solve this problem, because as has been mentioned above, that point can be, in practice, 6 to 10 feet from any temperature controller. Such an arrangement leads, with the low flow rates typically used in these surgical methods, to "transportation lags" which render stable feedback control difficult to achieve under major, rapid flow rate changes which are typically required by these endoscopic and laparoscopic surgical procedures. Thus, the gas reaches the patient at a temperature much lower than the desirable 36°C-38°C.

Insufflation gases typically are delivered extremely dry. In accordance with Food and Drug Administration guidelines, medical grade carbon dioxide, the most prevalent gas used for laparoscopy, contains 200 parts per million or less of water vapor. The extreme lack of moisture in the insufflation gas can lead to drying of exposed tissue surface within the abdomen, and to the possibility of adhesion formation within the peritoneal cavity Corfman, RC., Clinical Consultations in Obstetrics and Gynecology, 1:41-49 (1989)). With previous insufflation systems, frequent irrigation of the peritoneal cavity was required to limit adhesion formation.

The Douglas *et al*. patent discloses humidifying gas prior to administration to the patient. Its method and device to achieve this objective, however, does not allow for a number of important thermodynamic and psychrometric effects. For instance, it is not effective to thermally condition and then to humidify the gas (or visa versa) in a serial order (*see*, for example, Chapter 5, Psychrometrics, ASHRAE Handbook, Fundamentals, Section I, (The American Society of Heating, Refrigerating and Air Conditioning Engineer Inc., pp. 5.1-5.10 (1981)). Because of the intrinsic nature of the dependency of water vapor pressure upon temperature, it is not possible to obtain useful levels of relative humidity and temperature with the Douglas *et al*. device.

It is known to filter insufflation gas to prevent inorganic particles such as metallic fillings or particles, rust, dust, and polymer particles from passing into the pneumoperitoneum (*see*, *e.g*., Ott, D.E.,J. Gynecol. Surg., 5:205-208 (1989)). The location and type of filter, however, are very important factors which will influence the effectiveness of the method. Filters having a pore size as small as 0.2 microns have been used in previous insufflation systems. These devices, however, utilize a filter material that is typically hydrophilic and when it becomes moist, loses its strength and some of its filtering effectiveness. These filters, because they are not hydrophobic, can lose their filtering capability by tearing under the water pressure caused by accidentally suctioning peritoneal or irrigation fluids.

The article "The persufflator: an insufflation device for laparoscopy and especially for CO2-laser-endoscopic surgery", Koninckx, Human Reproduction, Vol. 6, No. 9, pp1288-1290, 1991 discloses a new insufflation device for laparoscopic surgery which permits the persufflation of the abdominal cavity with humidified CO₂ at 37°C.

AU 74,564/74 discloses a medical humidifier for use during artificial respiration or anaesthesis. The humidifier also has a heating means.

Typically, insufflator and other prior art insufflation gas conditioning methods utilize conventional, 120 volt, alternating current power commonly available in all operating rooms. There are two disadvantages to this power source. The number of devices requiring such power during surgery has become very large in recent years, which reduces space near the operating table and creates a tangle of power cords which compete for outlets and sometimes interfere with operating room procedures. Secondly, despite advanced grounding and isolation device and hospital procedures, there remains a finite probability of accidentally causing dangerous, sometimes lethal, patient shock from the 120 AC voltage.

Thus, previous devices attempting to provide conditioned gas to a patient have had significant problems and limitations. Accordingly, there is a great need for a method and apparatus for the conditioning of insufflation gas with appropriate physiological temperature, humidity and purity suitable for direct introduction to the peritoneal cavity in a manner which overcomes the limitations of previous systems.

### SUMMARY OF THE INVENTION

The disadvantages of the prior art are overcome by a high-efficiency apparatus for heating, humidifying and filtering gas, thus allowing the gas to be delivered to the patient at an accurate temperature while also properly humidified and filtered and without the use of AC voltage. An efficient, compact means of providing heated and humidified gas to a patient is provided such that heat loss in transfer of the gas is minimized, and thus, the gas is actually delivered to the patient within a desired predetermined temperature range.

Thus, a principle general object is to provide a compact, high efficiency, lightweight apparatus for heating and humidifying gas from a commercial insufflator that can be positioned immediately adjacent to the patient so that the gas exiting the apparatus is immediately delivered into the body cavity of the patient, and that has an efficient means for simultaneously heating the gas to a predetermined, preset temperature and humidifying the gas.

An apparatus for treating gas prior to the use of the gas in a medical procedure involving a patient is provided the gas being received into the apparatus from an insufflator which receives gas from a gas source, and the gas exiting the apparatus being in flow communication with a means for delivering the gas to the interior of the patient, wherein the gas is pressure- and volumetric flow rate-controlled by the insufflator, comprising a housing having an inlet and an outlet; means for communicating the outlet of the insufflator with the inlet of the housing; a chamber within the housing and having an entry port and an exit port, the entry port of the chamber being in flow communication with the inlet of the housing; a humidification means in the chamber that is in the path of travel of the gas through the chamber; heating means disposed within the humidification means for heating the gas to a predetermined temperature; means in the housing adjacent the exit port of the chamber for sensing the temperature of the gas; and means connected to the sensing means for controlling the heating means, whereby upon the determination by the sensing means of the temperature of the gas being at a predetermined level, the controlling means regulates the amount of heat applied by the heating means to the gas within the chamber. Additionally provided is a source of power for the controlling means. Also provided is the above apparatus wherein the source of power is a battery.

Another object is to provide an apparatus having a chamber having a high efficiency humidification bed and that simultaneously provides a filtration for the gas, wherein also the heating means is disposed within the humidification means. Accordingly, the apparatus provides for heating and humidifying gas for use in a medical procedure, comprising a chamber having an entry port and an exit port; humidification means within the chamber that is in the path of travel of the gas through the chamber; and means disposed within the humidification means for heating the gas to a predetermined temperature. The humidification means can comprise a volume of water and have the heating means disposed within or around the water.

A further object is to provide an apparatus further having a second means in the chamber for filtering the heated and humidified gas.

Still another object is to provide methods for treating gas that has been humidified and heated to a predetermined temperature such that the patient can receive gas that is within 2°C of the predetermined temperature.

### BRIEF DESCRIPTION OF THE DRAWINGS

Figure 1 is a schematic view of a gas treatment apparatus embodying the present invention, the apparatus being connected to an insufflator at one end and a means for delivering the gas to a patient at the opposite end, and being broken away at the heating and humidifying chamber to show the heating element within the chamber;
Figure 2 is a cross-sectional view of the healing and humidifying chamber of the present invention; and
Figure 3 is a typical wiring schematic of the means for sensing the temperatures within the chamber and controlling the heating element within the chamber.

### DESCRIPTION OF THE PREFERRED EMBODIMENTS

The present invention may be understood more readily by reference to the following detailed description of specific embodiments and the Examples and Figures included therein. Referring now to the drawings, it will become apparent that the important features of the present invention include the provision of a method for heating, humidifying and filtering gas prior to delivery to a patient such that the gas reaches the patient within 2°C of a predetermined temperature.

An apparatus for treating gas prior to the use of the gas in a medical procedure involving a patient, the gas being received into the apparatus from an insufflator which receives gas from a gas source, and the gas exiting the apparatus being in flow communication with a means for delivering the gas to the interior of the patient, wherein the gas is pressure- and volumetric flow rate-controlled by the insufflator, comprises a housing having an inlet and an outlet; means for communicating the outlet of the insufflator with the inlet of the housing; a chamber within the housing and having an entry port and an exit port, the entry port of the chamber being in flow communication with the inlet of the housing; a humidification means in the chamber that is in the path of travel of the gas through the chamber, heating means disposed within the humidification means for heating the gas to a predetermined temperature; means in the housing adjacent the exit port of the chamber for sensing the temperature of the gas; and means connected to the sensing means for controlling the heating means, whereby upon the determination by the sensing means of the temperature of the gas being at a predetermined level, the controlling means regulates the amount of heat applied by the heating means to the gas within the chamber. Additionally provided is a source of power for the controlling means. Also provided is the above apparatus wherein the source of power is a battery.

As used in the claims, "a" can mean one or more.

As used herein, "a predetermined temperature" is one that has been preset and is not altered during a procedure. For laparoscopic procedures, the desireable predetermined temperature is physiological body temperature, i.e, approximately 36-38°C.

As used herein, any apparatus "immediately adjacent" to a patient or an object indicates a position sufficiently physically close in proximity to the patient or object such that gas at a temperature in the apparatus will not lose more than 2°C while traveling from the apparatus to the interior of the patient or object Such a distance would be, for example, from about 0-25,4 cm (0-10 inches), preferably from 0 to 10 cm, and more preferably from 0 to 3 cm. Thus, the heater itself, within the chamber of the apparatus, is immediately adjacent to the patient.

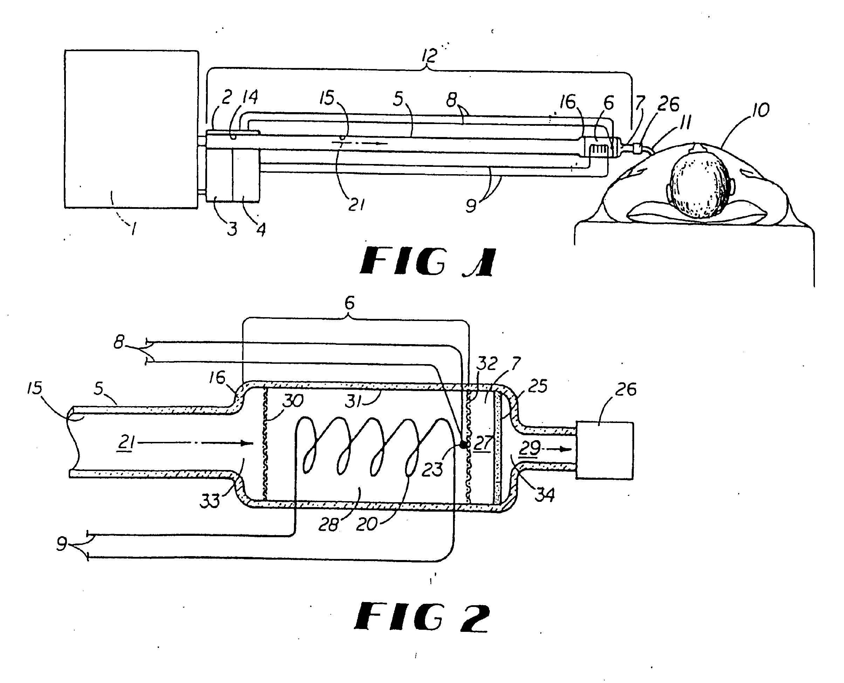

A system for conditioning gas is illustrated in Fig. 1, in which a patient 10 is shown lying on a bed or other support at a distance from a commercially available insufflator 1 (e.g., Storz Model 26012) which receives gas from a source such as a cylinder (not shown). The apparatus **12** of the present invention is located between the insufflator **1** and a gas delivery device **11**, such as a trocar or a needle, inserted into patient **10**, in flow communication with both the insufflator **1** and the patient **10.**

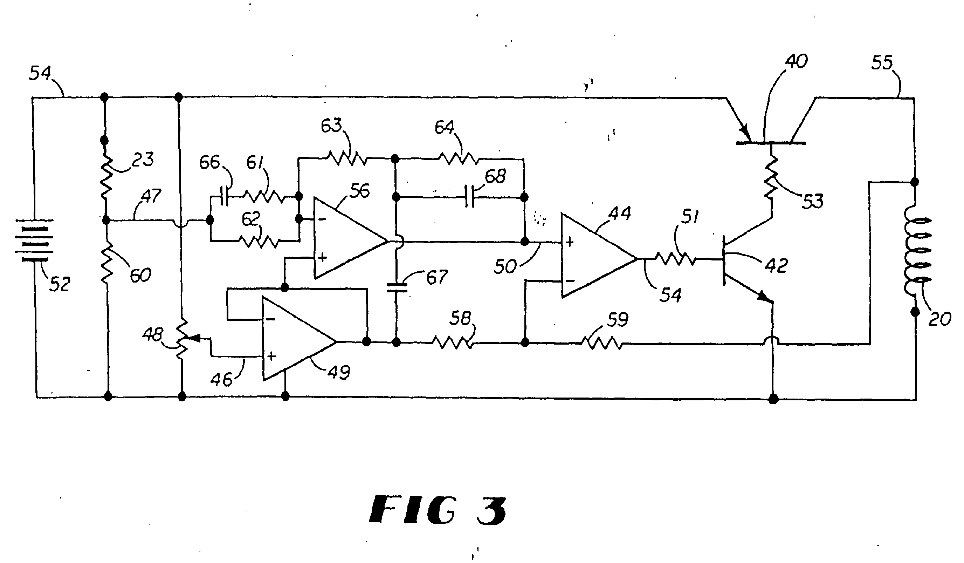

Fig. 1 illustrates a simplified version of the apparatus. The insufflator **1** is closely connected to an optional container **2,** which serves to enclose a battery power supply **3,** and an electronic temperature control circuit **4** (*see* Fig. 3). A preferred embodiment provides for the power to be activated automatically by the attachment of container **2** to insufflator **1,** such that heating can begin prior to activation of insufflator **1.** An optional interior passage **14** within container **2** permits gas **21,** which is pressure and flow regulated by insufflator **1,** to pass through it and into a lumen **15** of a gas transfer tube **5** which is connected to housing **16** such that lumen **15** is in flow communication with chamber **6**. In an alternative embodiment, interior passage **14** can be designed to loop into control circuit **4** within container **2** before flowing into gas transfer tube **5.** Such an arrangement allows heat exchange to occur between the relatively cool gas **21** within interior passage **14** and the relatively warm control circuit **4**, thereby cooling control circuit **4** and preheating gas **21**. In this alternative embodiment, interior passage **14** passes proximal enough to control circuit **4** to allow heat exchange to occur.

The gas transfer tube **5** is preferably flexible and sufficiently long " to permit the insufflator **1** and power supply **3** and control circuit **4** within container **2** to be placed at a convenient distance from the patient **10** undergoing laparoscopic surgery while housing **16** can be placed immediately adjacent to the patient **10.** Gas delivery device **11,** which inserts into patient **10,** preferably does not cause the gas to travel more than about 25,4 cm (10 inches), more preferably not more than about **10** centimeters, and preferably between about 0 and **3** centimeters, from housing **16,** before it reaches the interior of patient **10.** The purpose of this arrangement is to allow gas to be delivered to the patient **10** while still at a temperature sufficiently close to physiological interior body temperature. That is, the thermodynamic cooling of medical gases in transit to the patient is prevented, because a highly efficient heating and humidifying chamber **6** is provided that, because of its efficiency, can be quite compact and thus be positioned very near to patient **10.** In a preferred embodiment, heater/humidification chamber **6** is so compact and lightweight that the operator can hold chamber **6** to deliver gas. The gas transfer tube **5** and chamber **6** can be reused but are preferably disposed after single usage.

Once the insufflator **1** is activated, it receives gas from the gas supply cylinder and regulates the pressure and flow rate of the gas, both of which can be adjusted by the operator. Insufflator gas **21** then flows the full length of transfer tube 5 within lumen **15** where it enters heater/humidification chamber **6** at entry port **33,** crosses optional gas permeably membrane **30** and enters enclosure **31,** where it comes into contact with electrical heating element **20** and water-containing humidifying bed **28,** shown in Fig. 2. The pressure and volumetric flow rate are controlled by adjusting the insufflator controls (not shown). In chamber **6,** insufflator gas **21** is simultaneously heated and humidified to the proper physiological range by regulation of heater element **20** such that the temperature of gas **27** exiting chamber **6** is within a preselected physiological temperature range (preferably 36° to 38°C). The water containing humidifying bed **28** provides humidification within chamber **6** such that gas **27** exiting chamber **6** is humidified. After the insufflator gas **21** has been temperature/humidity conditioned in chamber **6**, it exits enclosure **31** (as gas **27**) through optional gas permeable membrane **32,** and enters chamber **7** where it passes through a high efficiency filter **25.** The gas **27** then exits chamber **7** through exit port **34** (as gas **29**) and into connector **26,** which can attach to a conventional gas delivery device **11** appropriate for the specific medical procedure for immediate delivery of gas **29** into patient **10.**

The heater element **20** of the heater/humidification chamber **6** receives its power from the battery power supply **3** and control circuit **4** by a flexible power wire pair **9** which can be attached to the outside, or be embedded within, the wall of the gas transfer tube **5.** Alternatively, gas transfer tube **5** can be provided with an additional lumen through which power wire pair **9** can pass.

Feedback temperature regulation of the heater/humidification chamber **6** is accomplished by detecting the temperature of gas **21** at the exit end of enclosure **31** by temperature sensing device **23** and the signal representing this temperature by signal transfer wire pair **8** to control circuit **4** of container **2.** As with power wire pair **9,** signal transfer wire pair **8** can be attached to the outside, or be embedded within, the wall of gas transfer tube **5,** or gas transfer tube **5** can be provided with an additional lumen through which signal transfer wire pair **8** can pass.

The temperature control circuit **4** is shown in detail in Fig. 3. The circuit **4** is powered by batteries **52** of battery power supply **3** located in container **2** and provide the circuit with battery voltage **54**. The use of a low voltage DC power source in this invention enables the apparatus to be independent of conventional AC electrical wiring of the hospital or clinic where the laparoscopic procedure is being carried out, which results in simple and quick installations and reduced wire clutter in the neighborhood of the surgery. Additionally, such low voltage DC power assures that the invention will be intrinsically safe from accidentally delivering dangerous electrical shocks to the patient or healthcare personnel. A self-contained battery having a voltage of from about 1.6 to about 3.0V is preferred, with a battery having voltage of from about 2.6 to about 3.0V being even more preferred (for example, Ray-o-Vac D cell, Ray-o-Vac Corp. Madison, Wisconsin).

Optimal temperature of gas **29** that enters patient **10** is at or near physiological interior body temperature (i.e., approximately 36°C to 38°C), or within 2°C of physiological body temperature, or even more preferably, within 0.5°C thereof. The temperature of the heated and humidified gas **27** is detected by a temperature sensing device **23.** As the temperature of temperature sensing device **23** changes, so does its electrical resistance, which in turn causes the current flow through it and resistor **60** to change, and therefore, the sense voltage **47** (V47) to be altered according to the proportion of the resistance of temperature sensor **23** to the total of the resistance of temperature sensor **23** plus that of resistor **60.**

Power transistor **40** (for example, MJE 2955, Motorola, Phoenix, Arizona) controls the time average voltage across electrical heater element **20.** Power transistor **40** is itself controlled by transistor **42,** whose base current is determined by the output voltage of the operational amplifier **44** (for example LM358, National Semiconductor, Inc., Santa Clare, California). The operational amplifier **44,** resistors **51, 53, 58, 59,** heater element **20,** and transistors **40** and **42** function as an inner feedback loop such that heater voltage **55** (V55) is given by: V55 = V50 + (R59/R58) (V50-V46), wherein V50 is voltage **50,** R59 is resistance at resistor **59** and R58 is resistance at resistor **58.** Variable potentiometer **48** provides the variable set point of the temperature controller and generates the variable reference voltage **46** (V46), which has a value between zero and battery voltage **54.** Operational amplifier **49** is a unity gain buffer which insures the voltage **46** is independent of other circuit variable.

The sense voltage **47** (V47) and the reference voltage **46** (V46) are inputs to operational amplifier **56,** which in turn outputs a voltage **50** (V50). Operational amplifier **56,** resistors **61, 62, 63** and **64** and capacitors **66, 67** and **68** act as two lead compensation networks to ensure a stable feedback system. The effect of this circuit is to cause the heater element voltage **55,** at zero frequency, to be given by: V55 = V46 + (1 + R59/R58) ((R63 + R64)/R62) (V47 - V46), wherein R63, R64 and R62 are resistance at resistor **63, 64** and **62,** respectively.

The location of container **2** in this preferred embodiment was selected to use the insufflator for access convenience and for physical support. Since no gas processing occurs in container **2,** the existence and location of container **2** is arbitrary and, if it is used, it can be placed at any position along gas transfer tube **5.** If container **2** is not used, battery power supply **3** and control circuit **4** can be placed at any convenient point(s) along gas transfer tube **5.**

Fig. 2 gives the specific configuration of the heating/humidification method and chambers **6** and **7.** Insufflator gas **21** enters chamber **6** from lumen **15** through entry port **33.** Gas **21** then crosses optional gas permeable membrane **30** to enter enclosure **31,** which contains a humidification bed **28** comprising a porous bed or reservoir of entrapped water, the water preferably being sterile. Humidification bed **28** is preferably composed of a water-retaining material which has been infused with water. The material can be any desirable water-retaining material, preferably a borosilicate-type material (e.g., Type A/E Glass Fiber Filters or Extra Thick Glass Fiber Filters, Gelman Sciences, Ann Arbor, Michigan). The pore size of the selected material should be chosen according to a balance of Water retention capabilities and filtering capabilities. The larger the pore size, the more water retention for humidification. However, in some embodiments, humidification bed **28** also serves to filter gas **21,** and the pore size chosen will depend upon the adequacy of filtration desired, as well as the presence or absence of filter **25** in chamber **7.** In the presence of filter **25** in the apparatus, the pore size of the material of humidification bed **28** can be chosen for water retention capabilities only, e.g., about **1** micron. Furthermore, when a water-retaining material infused with water comprises humidification bed **28,** gas permeable membranes **30** and **32** are not necessary.

Embedded in humidification bed **28** is an electric resistance heater element **20** which heats humidification bed **28** to a temperature that is predetermined, i.e., preset within control circuit **4.** Electric current is supplied to the heater element **20** via power wire pair **9,** from battery power supply **3,** located in container **2.** A temperature sensing device **23,** such as a thermistor (for example, Thermometrics Series BR23, Thermometrics, Inc., Edison, New Jersey), is located just prior to or immediately after optional gas permeable membrane **32** to sense the temperature of gas **21** as it exits enclosure **31,** and is used to generate a temperature indicating signal which is transferred to the feedback control circuit **4** via signal transfer wire pair **8.** It is preferable that the temperature sensor **23** be accurate to within about 0.1°C It is vital to the present invention that the temperature is sensed after it has been humidified so that any change in the temperature of gas **21** as it is humidified is corrected at that point in the apparatus. Details of power supply **3** and electronic control circuit **4** are shown in Fig. 3. A preferred range of humidification is from about 85-98% humidity.

The temperature- and humidity-conditioned gas **27** enters chamber **7,** wherein it passes through an optional high efficiency, hydrophobic filter **25** (for example Gelman Sciences Metricel M5PU025) having a pore size preferably small enough to exclude all solid particles and bacterial or fungal agents that may have been generated in the gas supply cylinder, insufflator **1,** or any location in the invention, including heater/humidifier bed **28** (i.e., 0.5 micron or less and preferably about 0.3 micron). A preferable filter is a hydrophobic filter, such as a glass fiber-type filter, e.g., Metrigard (Gelman Sciences). Other suitable filters include polysulfone (Supor, HT Tuffrin, Gelman Sciences) and mixed cellulose esters (GN-6 Metricel, Gelman Sciences), for example. Decreasing the pore size of filter **25** below 0.3 micron causes a concomitant increase in pressure drop of gas **29,** and thus flow rate is reduced significantly. If the medical procedure to be performed requires a relatively high pressure and/or flow rate of gas to the patient, such as laparoscopy, filter **25** pore size should preferably not decrease below 0.3 micron. The diameter of filter **25** should be chosen to fill the width of chamber **7** such that all gas passes through filter **25.** A hydrophobic filter is preferable to a hydrophilic one, as a hydrophobic filter is less likely to tear under water pressure caused by accidentally suctioning peritoneal or irrigation fluids.

The filtered and temperature/humidity conditioned gas **29** passes directly to a connector **26,** designed to attach to a conventional trocar used to inflate the peritonea or other such gas delivery device for the particular medical procedure. Housing **16** is preferably placed such that exit port **34** is within about 0-25,4 cm (0 to 10 inches) preferably within about 0 to 10 centimeters from patient **10,** and more preferably within about 1 to 3 centimeters. Thus, the gas **29** that is now heated, humidified and filtered passes immediately into the delivery device **11** and into the interior of patient **10,** while still maintained at the desired temperature, or at least within about 2°C of it, and preferably within about 0.5°C, because the invention is of such high efficiency and is designed to allow the heating to occur just proximal to the patient.

The length and width of chamber **6** can be varied such that proper heating and humidification still occur. As an example, an average chamber **6** will contain from about 3 to about 6 cubic centimeters of water, and it is desireable that gas have a dwell time within the chamber of at least approximately 0.01 to 0.5 second. Thus, the desireable width and diameter of the chamber will also be dependent upon the rate of gas flow from insufflator **1,** which is usually from about 1-10 liters/minute, and upon the pressure desired to be maintained, which is affected more by diameter of chamber **6** than by length. A person of skill in the art, given the teachings and examples wherein, can readily determine useful dimensions for chamber **6** without undue experimentation. It should also be noted, however, that upon activating the apparatus or changing the demand on the apparatus (e.g., flow rate or pressure), there is a lag time of milliseconds for sensing the temperature of gas and adjusting the heating to achieve the proper gas temperature. Such a fast start-up time is extremely beneficial. Furthermore, approximately the first 12 to 15 cubic centimeters of gas leaving the apparatus after it is activated are cooler than the predetermined temperature.

The apparatus provides for heating and humidifying gas for use in a medical procedure, comprising a chamber having an entry port and an exit port; humidification means within the chamber that is in the path of travel of the gas through the chamber; and means disposed within the humidification means for heating the gas to a predetermined temperature. The humidification means can comprise a volume of water and have the heating means disposed within or around the water, as described above. The water can be contained within water-retaining material such as a borosilicate-type material, as described above. Additionally, a filtering means can be provided, by the humidification bed and/or by an additional filter within the chamber. Filter sizes are preferably as described above.

A method of providing heated, humidified gas into a patient for an endoscopic procedure comprises the steps of directing pressure- and volumetric flow rate- controlled gas, received from an insufflator into a chamber having a means for heating the gas to a temperature within a predetermined range and a means for humidifying the gas and being disposed immediately adjacent to the patient, wherein the chamber is in flow communication with and immediately adjacent to a means for delivering the gas to the interior of the patient; sensing the temperature of the gas as it exits the chamber to determine if it is within the predetermined range; and actuating the heating means if the temperature of the gas is without the predetermined range; humidifying the gas within the chamber, and flowing the gas into the delivering means such that the gas enters the patient humidified and having a temperature within 2°C of the predetermined temperature and thus providing the gas. The heating means and the humidifying means can heat and humidify the gas simultaneously. The gas can be filtered as described above, as the gas is heated and humidified and/or immediately after it is heated and humidified. The heating means can be controlled by a power source comprising a self-contained battery of voltage in the range of about 1.6 -3.0V. An even more preferable range is about 2.6-3.0V.

Also provided is a method of treating gas for use in a medical procedure comprising the steps of directing the gas into the inlet of a chamber having an inlet and an outlet and having a means for beating the gas to a temperature within a predetermined range and a means for humidifying the gas, such that the gas can be heated and humidified simultaneously; sensing the temperature of the gas as it exits the chamber to determine if it is within the predetermined range; and actuating the heating means if the temperature of the gas is without the predetermined range; humidifying the gas within the chamber, and receiving the heated and humidified gas at the predetermined temperature from the outlet of the chamber, thus treating the gals. The humidifying means can serve also to filter the gas, such as by water and/or by a water-retaining material containing water, as described above. Additionally, the heated and humidified gas can be filtered a second time through a second filter within the chamber.

The method and apparatus can be utilized for many medical procedures requiring the provision of heated and humidified gas. The optional filtration may also be utilized according to the sterility of gas required for the procedure. The gas is chosen according to the procedure to be performed and can be any medically useful gas, such as carbon dioxide, nitrous oxide, argon, helium, nitrogen and room air and other inert gases. Preferable gases for endoscopy are carbon dioxide and nitrous oxide. A combination of the above gases can also be used, i.e., 100% of a single gas need not be used. In particular, the compact size of the apparatus and its use of a battery as a source of power make the invention portable and thus suitable for uses requiring portability. The gas delivery device that provides the direct contact to the patient should be selected according to the medical procedure to be performed as known to those skilled in the art.

The present invention is more particularly described in the following examples which are intended as illustrative only since numerous modifications and variations therein will be apparent to those skilled in the art.

### EXAMPLES

The following table gives the temperature and humidification levels resulting from three different heater elements, and water retention porous bed sizes. Many other configurations and combinations have been tested and found satisfactory.

**TABLE 1: Operating Characteristics**

| **Porous Bed Characteristics(1)** | | **Heater Element Type(2)** | **Flow Rate L/m** | **Relative Humidity Range% (3)** | **Temperature Range °C(4)** |
|---|---|---|---|---|---|
| **Size** | **Material** | | | | |
| 3x16 | AT4 | 1-19L | 1.5 | 75-82 | 24-48 |
| | | | 9.0 | | 24-36 |
| 2x8.6 | T4 | 0.8-15S | 1.0 | 60-70 | 22-45 |
| | | | 5.0 | 27-69 | 20-35 |
| 16x19 | GS A/D | 0.8-15S | 1.0 | 75-85 | 30-40 |
| | | | 9.0 | 85-95 | 24-32 |

| | | | | | |
|---|---|---|---|---|---|
| (1) Size: 1st number=bed length in cm, 2nd mumber=bed diameter in mm. Material: Manufacturer's code: AT4, Arbor Tech, Inc, Ann Arbor, Michigan and GS A/D, Gelman Sciences, Ann Arbor, Michigan. (2) Type: 1st number=element resistance in ohms, 2nd number=wire length in cm. Letter=L designates longitudinal, S designates spiral. (3) Relative humidity was dependent upon selected temperature of the gas and selected gas flow rate and was not independently controlled. (4) The listed temperatures indicate the range of temperatures from which the predetermined temperature was selected. Each selected temperature was maintained within ± 2.0°C, except for the short transient time period after start up of the apparatus or a new gas flow rate demand was placed upon the apparatus. | | | | | |

Although the present process has been described with reference to specific details of certain embodiments thereof, it is not intended that such details should be regarded as limitations upon the scope of the invention except as and to the extent that they are included in the accompanying claims.

## Claims

1. A method of treating gas (21) for use in an endoscopic procedure comprising the steps of:
a) directing the gas along a single path, wherein the step of directing the gas along a single path comprises;
(i) directing the gas (21) received from an insufflator (1) into the inlet of a chamber (6) having an inlet and an outlet and having a means for heating (20) the gas to a temperature within a predetermined range and a means for humidifying (28) the gas such that the gas can be heated' and humidified simultaneously;
ii) setting the set point of a variable temperature controller of the heating means (20) to generate a variable reference voltage,
iii) sensing the temperature of the gas as it exits the chamber (6) by using a temperature sensor (23) sensing a sense voltage to determine with respect to said reference voltage if said temperature is within the predetermined range; and
iv) actuating the heating means (20) if the temperature of the gas is without the predetermined range;
v) humidifying the gas (28) within the chamber; and
vi) receiving the heated and humidified gas (27) at the predetermined temperature from the outlet of the chamber, thus treating the gas,
wherein the endoscopic procedure is a laparoscopic procedure and the predetermined temperature is physiological body temperature.

2. The method of claim 1, wherein the means for humidifying the gas (28) also serves to filter (21) the gas.

3. The method of claim 2, further comprising the step of filtering the heated and humidified gas a second time through a second filter (25) within the chamber (7), thereby receiving heated, humidified and filtered (29) gas from the outlet of the chamber.

4. The method of claim 1, wherein the heating means (20) and the humidifying means (28) heat and humidify the gas simultaneously.

5. The method of claim 4, further comprising the step of filtering the gas (21) as it is heated and humidified.

6. The method of claim 5, further comprising the step of filtering the gas a second time (25) after it is heated and humidified.

7. The method of claim 1, wherein the heating means (20) is controlled by a power source comprising a self-contained battery (52) of voltage (54) in the range of about 1.6 - 3.0.

8. The method of claim 1, wherein a communicating means (5), communicating an inlet of the insufflator (1) with the inlet of a housing (16), is disposed distal to the insufflator (1).

9. The method of claim 1, wherein a portion of a communicating means (5), communicating an inlet of the insufflator (1) with the inlet of a housing (16), is positioned proximal to a controlling means (4), whereby the gas cools the controlling means (4) and the controlling means (4) preheats the gas.

10. The method of claim 1, wherein the gas is carbon dioxide or nitrous oxide.

## Patentansprüche

1. Verfahren zur Behandlung eines Gases (21) zum Gebrauch in einem endoskopischen Verfahren, das folgende Schritte aufweist:
a) Leiten des Gases entlang eines einzigen Pfades, wobei der Schritt des Leitens des Gases entlang eines einzelnen Pfades umfasst;
i) Leiten des Gases (21), erhalten von einem Insufflator (1), in den Einlass einer Kammer (6), die einen Einlass und einen Auslass besitzt und die ein Heizmittel (20) zum Aufwärmen des Gases auf eine Temperatur innerhalb eines vorbestimmten Bereichs und ein Befeuchtungsmittel (28) zum Befeuchten des Gases besitzt, derart, dass das Gas gleichzeitig aufgewärmt und befeuchtet werden kann;
ii) Einstellen des Einstellpunktes eines variablen Temperatursteuergerätes des Heizmittels (20), um eine variable Referenzspannung zu erzeugen,
iii) Messen der Temperatur des Gases, wenn es die Kammer (6) verlässt unter Verwendung eines Temperatursensors (23), der eine Messspannung misst, um mit Bezug auf die Referenzspannung zu bestimmen, ob die Temperatur innerhalb des vorbestimmten Bereichs liegt und
iv) Betätigen des Heizmittels (20), falls die Temperatur des Gases außerhalb des vorbestimmten Bereichs ist;
v) Befeuchten des Gases (28) innerhalb der Kammer; und
vi) Empfangen des erwärmten und befeuchteten Gases (27) bei der vorbestimmten Temperatur von dem Auslass der Kammer, die das Gas auf diese Weise behandelt hat,
wobei das endoskopische Verfahren ein laparoskopisches Verfahren ist und die vorbestimmte Temperatur die physiologische Körpertemperatur ist.

2. Verfahren nach Anspruch 1, wobei das Befeuchtungsmittel (28) zum Befeuchten des Gases auch zum Filtern (21) des Gases dient.

3. Verfahren nach Anspruch 2, das weiterhin den Schritt des Filterns des erwärmten und befeuchteten Gases ein zweites Mal durch ein zweites Filter (25) innerhalb der Kammer (7) umfasst, wodurch erwärmtes, befeuchtetes und gefiltertes Gas (29) von dem Auslass der Kammer empfangen wird.

4. Verfahren nach Anspruch 1, wobei das Heizmittel (20) und das Befeuchtungsmittel (28) das Gas gleichzeitig aufwärmen und befeuchten.

5. Verfahren nach Anspruch 4, das weiterhin den Schritt des Filterns des Gases (21), wenn es erwärmt und befeuchtet wird, umfasst.

6. Verfahren nach Anspruch 5, das weiterhin den Schritt des Filterns des Gases (25) ein zweites Mal, nachdem es erwärmt und befeuchtet ist, aufweist.

7. Verfahren nach Anspruch 1, wobei das Heizmittel (20) durch eine Spannungsquelle gesteuert wird, die eine Eigenbatterie (52) der Spannung (54) in dem Bereich von etwa 1,6 bis 3 Volt aufweist.

8. Verfahren nach Anspruch 1, wobei ein Kommunikationsmittel (5), das einen Einlass des Insufflators (1) mit dem Einlass eines Gehäuses (16) verbindet, distal zu dem Insufflator (1) angeordnet ist.

9. Verfahren nach Anspruch 1, wobei ein Bereich eines Kommunikationsmittels (5), das einen Einlass des Insufflators (1) mit dem Einlass eines Gehäuses (16) verbindet, proximal zu einem Steuermittel (4) positioniert ist, wobei das Gas das Steuermittel (4) kühlt und das Steuermittel (4) das Gas vorwärmt.

10. Verfahren nach Anspruch 1, wobei das Gas Kohlendioxid oder Distickstoffmonoxid ist.

## Revendications

1. Procédé de conditionnement de gaz (21) pour utilisation dans une méthode endoscopique comprenant les étapes qui consistent à :
a) acheminer le gaz par un chemin unique, ladite étape d'acheminement du gaz par un chemin unique comprenant les étapes consistant à ;
(i) acheminer le gaz (21) reçu d'un insufflateur (1) à l'entrée d'une chambre (6) comprenant une entrée et une sortie et comprenant un moyen pour chauffer (20) le gaz à une température comprise dans une gamme prédéterminée de températures et un moyen pour humidifier (28) le gaz de manière à ce que le gaz puisse être chauffé et humidifié simultanément ;
(ii) établir le point de consigne d'un contrôleur de température variable associé au moyen de chauffage (20) pour générer une tension variable de référence ;
(iii) détecter la température du gaz quand il sort de la chambre (6) en utilisant une sonde de température (23) qui détecte une tension de mesure pour déterminer en fonction de ladite tension de référence si ladite température est comprise dans ladite gamme prédéterminée ; et
(iv) actionner le moyen de chauffage (20) si la température de gaz est hors de la gamme prédéterminée ;
(v) humidifier le gaz dans la chambre ; et
(vi) recevoir le gaz humidifié et chauffé (27) à la température prédéterminée de la sortie de la chambre, ayant de la sorte traité le gaz,
la méthode endoscopique précitée étant une méthode laparoscopique et la température prédéterminée étant la température physiologique du corps.

2. Procédé selon la revendication 1, dans ledit procédé, le moyen servant à humidifier le gaz (28) sert également à filtrer (21) le gaz.

3. Procédé selon la revendication 2, comprenant en outre les étapes consistant à filtrer le gaz chauffé et humidifié une deuxième fois à travers un deuxième filtre (25) dans la chambre (7), ce qui permet de la sorte de recevoir le gaz chauffé, humidifié et filtré (29) de la sortie de la chambre.

4. Procédé selon la revendication 1, dans ledit procédé, le moyen de chauffage (20) et le moyen d'humidification (28) chauffent et humidifient le gaz simultanément.

5. Procédé selon la revendication 4, comprenant en outre l'étape consistant à filtrer le gaz (21) en même temps qu'il est chauffé et humidifié.

6. Procédé selon la revendication 5, comprenant en outre l'étape consistant à filtrer le gaz une deuxième fois (25) une fois qu'il a été chauffé et humidifié.

7. Procédé selon la revendication 1, dans ledit procédé, le moyen de chauffage (20) est commandé par une source d'alimentation électrique comprenant une batterie autonome (52) dont la tension électrique (54) est comprise dans une gamme d'environ 1,6 - 3,0.

8. Procédé selon la revendication 1, dans ledit procédé un moyen de communication (5), faisant communiquer une entrée de l'insufflateur (1) avec l'entrée d'un logement (16), est disposé en position distale par rapport à l'insufflateur (1).

9. Procédé selon la revendication 1, dans ledit procédé une partie d'un moyen de communication (5), faisant communiquer une entrée de l'insufflateur (1) avec l'entrée d'un logement (16), est disposée en position proximale par rapport à un moyen de commande (4), ce par quoi le gaz refroidit le moyen de commande (4) et le moyen de commande (4) préchauffe le gaz.

10. Procédé selon la revendication 1, ledit gaz étant le dioxyde de carbone ou l'oxyde nitreux.
